# EUROPÄISCHE PATENTSCHRIFT

(11) **EP 4 393 455 B1**
(45) Veröffentlichungstag und Bekanntmachung des Hinweises auf die Patenterteilung: **11.11.2026**
(21) Anmeldenummer: 23220708.4
(22) Anmeldetag: 29.12.2023
(51) Int. Cl.: A61F 5/02

(54) **ORTHESE MIT EINEM RUMPFABSCHNITT ZUR BEHANDLUNG VON WIRBELSÄULENSTELLUNGEN, DIE VON EINER NORMALSTELLUNG ABWEICHEN**
ORTHOSIS WITH A FUSELAGE SECTION FOR TREATING SPINAL COLUMN POSITIONS THAT HAVE A DEVIATION FROM A NORMAL POSITION
ORTHÈSE COMPRENANT UNE PARTIE DE COQUE POUR LE TRAITEMENT DE POSITIONS RACHIDIENNES S'ÉTENDANT À PARTIR D'UNE POSITION NORMALE

(30) Priorität: 29.12.2022 DE 102022135000
(43) Veröffentlichungstag der Anmeldung: 03.07.2024
(73) Patentinhaber: Eisert, Claudia, 64367 Mühltal (DE)
(72) Erfinder: EISERT, Claudia, 64367 Mühltal (DE); LOCHNO, Martina, 73230 Kirchheim unter Teck (DE); RÖDER, Uwe, 73035 Göppingen (DE)
(74) Vertreter: Habermann Intellectual Property Partnerschaft von Patentanwälten mbB

(56) Entgegenhaltungen:
- EP-A1- 2 967 964
- EP-A1- 3 861 967
- KR-B1- 102 047 060
- US-A1- 2019 380 408
- US-A1- 2020 260 804

## Beschreibung

Die Erfindung betrifft eine Orthese mit einem Rumpfabschnitt zur Behandlung von Wirbelsäulenstellungen, die von einer Normalstellung abweichen, wobei der Rumpfabschnitt bei einer bestimmungsgemäßen Verwendung der Orthese einen Rumpf eines mit der Orthese behandelnden Trägers zumindest abschnittsweise umhüllt, und wobei der Rumpfabschnitt mindestens einen aus einem ersten elastischen textilen Gewebe hergestellten Halteabschnitt und mindestens einen ein zweites textiles Gewebe aufweisenden Funktionsabschnitt aufweist, wobei der mindestens eine Funktionsabschnitt eine Vorspannung aufweist, wobei eine aus der Vorspannung resultierende Kraft und/oder ein Kraftmoment bei der bestimmungsgemäßen Verwendung der Orthese auf den Rumpf des Trägers wirkt, wobei der mindestens eine Halteabschnitt und der mindestens eine Funktionsabschnitt überlappungsfrei miteinander verbunden sind und den Rumpfabschnitt bilden, wobei ein Randbereich des mindestens einen Funktionsabschnitts mit einem Randbereich des mindestens einen Halteabschnitts entlang einer Funktionsnaht miteinander verbunden ist, wobei die beiden längs der Funktionsnaht miteinander verbundenen Randbereiche des mindestens einen Funktionsabschnitts und des mindestens einen Halteabschnitts eine voneinander abweichende Vorspannung aufweisen, und wobei ein Umfangsrand eines Zuschnitts des mindestens einen Funktionsabschnitts in einem nicht vorgespannten Zustand mindestens abschnittsweise einen Randabstand zu einem Umfangsrand eines von den Zuschnitten der angrenzenden Halteabschnitten nicht bedeckten Funktionsabschnittsbereichs aufweist.

Eine Orthese ist ein medizinisches Hilfsmittel zur Beeinflussung der strukturellen und funktionalen Eigenschaften des neuromuskulären wie auch des skelettalen Systems eines Trägers der Orthese. Im Gegensatz zu Prothesen dienen Orthese nicht dem Ersatz fehlender Körperteile, sondern zur Unterstützung oder Beeinflussung von Körperteilen. Dabei werden Orthesen oftmals an den jeweiligen Körper eines Trägers angepasst und können zur Stabilisierung, Entlastung, Ruhigstellung, Führung oder Korrektur von Gliedmaßen und/oder des Rumpfs beitragen.

Orthesen können bei der Behandlung von Wirbelsäulenstellungen eingesetzt werden, die von einer Normalstellung abweichen. Als Skoliose wird beispielsweise eine dreidimensionale Verkrümmung der Wirbelsäule bezeichnet, wobei es zu Krümmungen und Verschiebungen der Wirbelkörper der Wirbelsäule in den drei Körperebenen, der Frontal-, der Sagittal- und der Transversalebene kommen kann. Dabei handelt es sich in vielen Fällen um eine seitliche Verkrümmung der Wirbelsäule, die oft von einem Verdrehen einzelner oder mehrerer Wirbelkörper begleitet wird. Die eigentliche Stützmuskulatur der Wirbelsäule ist dabei meist nicht oder nur unzureichend in der Lage, die von einer Normalstellung abweichende Lage der Wirbelkörper zu kompensieren und die Wirbelsäule selbst somit zu stabilisieren.

Skoliose zählt dabei zu den klassischen orthopädischen Erkrankungen. Während leichte Skoliosen erfolgreich mit Bewegungstherapien behandelt und auch gelindert werden können, erfordern besonders schwere Schiefstellungen, die beispielsweise die Herz-Lungen-Funktion beeinträchtigen, meist eine Behandlung durch chirurgische Eingriffe, um der Fehlstellung entgegenzuwirken. Ziel einer jeden Therapie ist dabei unabhängig von der im Einzelfall gewählten Form die Begradigung und gegebenenfalls eine Derotation der Wirbelsäule, und damit einhergehend die Stabilisierung und Verbesserung der möglicherweise eingeschränkten Herz-Lungen-Funktion und die Reduzierung der aus der Fehlstellung resultierenden Rückenschmerzen.

Aus der Praxis sind hierzu Orthesen mit einem den Rumpf des Trägers zumindest teilweise umhüllenden Halteabschnitt und einen Kräfte oder Kraftmomente auf den Rumpf des Verwenders bewirkenden Funktionsabschnitt bekannt. Durch die durch den Funktionsabschnitt auf den Rumpf bewirkten Kräfte oder Kraftmomente kann dabei einer bestehenden Verkrümmung und/oder einer Rotation der Wirbelsäule entgegengewirkt werden. Der Halteabschnitt kann, wie beispielsweise in der Druckschrift EP 3 861 967 A1 beschrieben, aus einem elastischen textilen Gewebe wie einem T-Shirt gebildet sein, das von dem Träger wie das entsprechende Kleidungsstück getragen werden kann. Der Funktionsabschnitt ist als auf das T-Shirt aufgenähte elastische Zugbänder ausgebildet, welche zwei Abschnitte auf der T-Shirt-Oberfläche miteinander verbinden. Durch die Vorspannung des Zugbandes werden die zwei Abschnitte, an denen das Zugband befestigt ist durch die aus der Vorspannung resultierenden Zugkraft zueinander gezogen, wodurch eine die Haltung korrigierende Kraft auf den Rumpf des Trägers ausgeübt wird. Somit können Zugkräfte und bei einer geeigneten Anbringung mehrerer Zugbänder auch Rotationskräfte auf den Rumpf ausgeübt werden.

Die aus der Praxis bekannten Orthesen sind dabei durch die aufgenähten oder anderweitig aufgebrachten Zugbänder mehrlagig ausgestaltet, wodurch der Tragekomfort erheblich reduziert wird. Weiterhin können derartige Orthesen durch die aus der Doppellagigkeit resultierenden Schichtdicke oftmals auch die Bewegung des Trägers unnötig einschränken und dadurch den Alltag behindern. Dabei ist der Erfolg einer derartigen konservativen Behandlung einer Skoliose mittels einer Orthese auch maßgeblich durch die Akzeptanz der Orthese durch den Träger bedingt. Durch eine geringe Akzeptanz des Trägers, durch beispielsweise einen schlechten Tragekomfort, kann es zu Therapieabbrüchen kommen, wobei häufig auf einen chirurgischen Eingriff zurückgegriffen wird, welcher den Leidensweg abkürzen und eine konservative Therapie überflüssig machen soll. Dies ist jedoch unvermeidlich mit erheblichen Risiken eines solchen invasiven Eingriffs verbunden.

Beispielsweise in KR 102 047 060 B1 oder US 2019/380408 A1 sind jeweils Orthesen gezeigt und beschrieben, bei denen ein Funktionsabschnitt einer Rumpfbekleidung aus einem elastischen textilen Material besteht, dessen Vorspannung höher als eine Vorspannung des für den angrenzenden Halteabschnitt verwendeten textilen Materials ist. Durch eine geeignete Ausrichtung des für den Funktionsabschnitt verwendeten elastischen textilen Materials, sodass in einer gewünschten Wirkungsrichtung eine größere Zugkraft des elastischen Materials als in einer anderen Richtung erzeugt wird, kann eine die Haltung korrigierende Kraft auf den Träger ausgeübt werden.

Als Aufgabe der vorliegenden Erfindung wird es deshalb angesehen, die bereits aus dem Stand der Technik bekannten Orthesen mit einem Rumpfabschnitt zur Behandlung von Wirbelsäulenstellungen, die von einer Normalstellung abweichen, zu verbessern und die Akzeptanz einer derartigen Behandlung durch einen erhöhten Tragekomfort zu verbessern.

Die Erfindung wird dadurch gelöst, dass der Randabstand in Richtung eines Verlaufs des mindestens einen Funktionsabschnitts größer, vorzugsweise um einen Faktor von 50% größer und besonders vorzugsweise um einen Faktor von 80% größer als in einer Richtung quer zu dem Verlauf des mindestens einen Funktionsabschnitts ist, wobei der Verlauf des Funktionsabschnitts als längste mögliche Mittenlinie mit einem gleichen Abstand zu den gegenüberliegenden Randbereichen des Funktionsabschnitts (3) definiert ist.

Die geometrische Korrektur der Wirbelsäulenkrümmung und/oder Wirbelsäulenrotation kann durch die erfindungsgemäße Orthese durch den Einsatz von gegebenenfalls multidirektionalen auf den Rumpf des Verwenders gerichteten Kräften oder Kraftmomenten verbessert werden. Die Übertragung der Kräfte oder Kraftmomenten kann dabei durch Haftreibung zwischen dem Rumpfabschnitt und der Haut des Trägers flächig auf den Rumpf übertragen werden. Um den Tragekomfort und damit die Toleranz des Trägers zu steigern bzw. beizubehalten und Therapieabbrüchen vorzubeugen ist die Orthese so ausgebildet, dass der mindestens eine Halteabschnitt und der mindestens eine Funktionsabschnitt überlappungsfrei miteinander verbunden sind. Eine Verbindung kann über verbindende Nähte erfolgen. Eine geringfügige Überlappung eines Halteabschnitts und eines Funktionsabschnitts entlang eines Nahtverlaufs, welche nur der Herstellung der betreffenden Naht dient, wird im Sinne dieser Erfindung als überlappungsfreie Verbindung angesehen. Diese überlappungsfreie Anordnung ermöglicht eine bessere Kontrolle der durch die Orthese übertragenen Kräfte, da keine Überlappung von flexiblen textilen Geweben mit unterschiedlichen Eigenschaften vorhanden ist. Weiterhin wird dadurch ebenfalls der Tragekomfort erhöht, da beispielsweise keine zusätzlichen Zugbänder benötigt werden, die in den Rumpfabschnitt eingearbeitet oder aufgesetzt werden müssen.

Dazu kann der mindestens eine Funktionsabschnitt an dem mindestens einen Halteabschnitt festgelegt werden und mit diesem beispielsweise vernäht sein, sodass aus den beiden Abschnitten ein flächiges flexibles textiles Gewebe entsteht. Durch geeignete Berechnungen und mit Hilfe von Erfahrungen können die für den therapeutischen Erfolg erforderliche Anordnung des mindestens einen Funktionsabschnitt sowie die Materialbeschaffenheit, der Zuschnitt, die Orientierung und die Größe des mindestens einen Funktionsabschnitts und die daraus resultierenden Kräfte oder Kraftmomente für jeden Einzelfall und unter Berücksichtigung deformationsspezifischer Kenngrößen festgelegt werden.

Dabei ist es vorteilhaft, wenn der mindestens eine Funktionsabschnitt eine von dem mindestens einen Halteabschnitt unterschiedliche Rückstellkraft aufweist, wobei als Rückstellkraft eine Kraft bezeichnet wird, welche bei einer Ausdehnung des Funktionsabschnitts oder des Halteabschnitts der Ausdehnung entgegenwirkt und den ausgedehnten Funktionsabschnitt oder Halteabschnitt in eine ungedehnte Formgebung zurückverformen will. Das Spektrum der Rückstellkraft eines textilen Gewebes kann vergleichsweise unterschiedlich vorgegeben sein und sich von dehnungsweich bis dehnungshart erstrecken. Die Rückstellkräfte können eine lineare oder beispielsweise eine exponentielle Zug-Dehnungscharakteristik aufweisen. Die von dem mindesten einen Funktionsabschnitt infolge seiner Dehnung erzeugten Rückstellkräfte und dadurch bewirkten und auf einen Träger der Orthese ausgeübten Zugkräfte können dabei durch die Ausgestaltung des Gewebes isotrop oder auch anisotrop ausgebildet sein. Indem ein Funktionsabschnitt entgegen der von dem textilen Gewebe erzeugten Rückstellkraft ausgedehnt und in seiner ausgedehnten Formgebung in den Rumpfabschnitt der Orthese eingefügt wird, erzeugt die Rückstellkraft eine Vorspannung und dadurch bewirkte Zugkräfte, welche bei einer bestimmungsgemäßen Verwendung auf den Träger der Orthese übertragen werden. Durch die Vorgabe oder Auswahl geeigneter textiler Gewebe, die eine für die Verwendung in der Orthese geeignete Rückstellkraft aufweisen, können unterschiedliche Vorspannungen bei Funktionsabschnitten und Halteabschnitten realisiert und in geeigneter Weise Kräfte auf einen Träger der Orthese ausgeübt, bzw. übertragen werden, die einer Wirbelsäulenfehlstellung oder -fehlhaltung entgegenwirken.

Neben dem hohen Tragekomfort ist es auch möglich, durch eine geeignete Ausgestaltung der verwendeten textilen Gewebe der Orthese wie auch durch eine Farbgebung eine Orthese zu realisieren, die nach außen nicht als solche erkannt wird. Dies kann insbesondere durch die einlagige Ausgestaltung und ohne die ansonsten verwendeten und eine Orthese als solche zu erkennen gebenden Zugbänder erreicht werden, sodass die Orthese nicht als Mittel zur therapeutischen Behandlung wahrgenommen wird, sondern wie ein übliches Bekleidungsstück.

Bei dem Einsatz von mehreren Funktionsabschnitten können die einzelnen Funktionsabschnitte voneinander unterschiedliche Zugkräfte aufweisen, sodass beispielsweise mehrere Funktionsabschnitte eine im Wesentlichen in eine Richtung resultierende Zugkraft aufweisen. Der mindestens eine Funktionsabschnitt kann dabei ein flächiges Gebilde sein oder auch streifenförmig ausgebildet sein.

Neben dem mindestens einen Halteabschnitt und dem mindestens einen Funktionsabschnitt kann der Rumpfabschnitt auch einen Kompressionsabschnitt aufweisen. Der Kompressionsabschnitt kann dabei durch seine Ausgestaltung eine allseitig wirkende Zugkraft aufweisen und somit den Rumpf durch Kompression stützen.

Bei einem Verbinden eines Funktionsabschnitts mit einem Halteabschnitt muss der Randbereich des Funktionsabschnitts gedehnt werden, so dass bei einem Verbinden der beiden Randbereiche entlang der Funktionsnaht beide Randbereiche gleich lang sind. Der vorgedehnte Randbereich und damit der ganze Funktionsabschnitt wird damit mit einer Vorspannung versehen, woraus bei einem Anziehen der Orthese und einer dadurch bewirkten weiteren Dehnung des Funktionsabschnitts durch den Träger die aus der Vorspannung resultierende Zugkraft auf den Träger übertragen wird. Die Vorspannung ist dabei unter anderem abhängig von der Art des textilen Gewebes des Funktionsbereichs, dem Ausmaß der Dehnung bzw. des Längenunterschieds der beiden zusammengefügten Randbereiche, sowie von weiteren Faktoren.

Auf diese beschriebene Weise ist es nicht nur möglich, einen Funktionsabschnitt mit einem Halteabschnitt zu verbinden, sondern es können weiterhin auch zwei Funktionsabschnitte miteinander verbunden werden. Weiterhin ist es auch möglich einen Funktionsabschnitt allseitig mit anderen Funktionsabschnitten und/oder Halteabschnitten zu verbinden.

Über die unterschiedlichen Dehnungseigenschaften der Funktionsabschnitte und der Halteabschnitte werden über die Oberfläche des Rumpfes verlaufende Zugkräfte erzeugt. Durch die bei der Herstellung der Orthese vorgegebene Vorspannung des mindestens einen und gegebenenfalls mehreren Funktionsabschnitte können die während des Tragens der Orthese auf den Träger ausgeübten Zugkräfte in vorteilhafter Weise verstärkt werden. Eine besonders einfache Weise, diese Vorspannung zu erzeugen und vorzugeben, kann über einen in geeigneter Weise vorgegebenen Zuschnitt des mindestens einen Funktionsabschnitts in einem nicht vorgespannten Zustand verwirklicht werden.

Beispielsweise kann der Zuschnitt eines nicht vorgespannten Funktionsabschnitts in Richtung seines Verlaufs und damit in Richtung der gewünschten Zugkräfte eine geringere Erstreckung aufweisen, als es durch die angrenzend angeordneten Halteabschnitte und den von diesen Halteabschnitten freigelassenen Bereich der Orthese vorgegeben ist. Anschließend muss der Zuschnitt des Funktionsabschnitts gedehnt und dadurch vorgespannt werden, um entlang eines Umfangsrands mit den angrenzenden Rändern der Halteabschnitte verbunden zu werden. Der gedehnte und dadurch vorgespannte Funktionsabschnitt kann sich nach dem Verbinden mit den angrenzenden Halteabschnitten nicht mehr vollständig zusammenziehen, da sich die Halteabschnitte nicht gleichermaßen zusammenziehen lassen und dem Zusammenziehen des Funktionsabschnitts entgegenwirken. Der Funktionsabschnitt wird in einem vorgespannten Zustand verbleiben und dadurch bei einer Verwendung der Orthese verstärkt Kräfte und Momente auf den Träger der Orthese ausüben.

Durch die geeignete Vorgabe eines Randabstands des nicht vorgespannten Zuschnitts des mindestens einen Funktionsabschnitts kann in einfacher Weise eine an den jeweiligen Träger angepasste Vorspannung vorgegeben werden. Der Randabstand kann hinsichtlich seiner Größe entlang eines Umfangsrands des Funktionsabschnitts unterschiedlich vorgegeben und an den betreffenden Träger und die gewünschte Wirkung der Orthese angepasst sein. Der Randabstand kann sich über mehrere Abschnitte entlang des Umfangsrands erstrecken und darauf begrenzt sein, sodass in anderen Abschnitten entlang des Umfangsrands kein Randabstand vorgegeben wird und der Funktionsabschnitt im nicht vorgespannten Zustand ohne einen Randabstand an die angrenzenden Randbereiche der Halteabschnitte angepasst ist. Der Randabstand kann entlang des Umfangsrands des Funktionsabschnitts zumindest innerhalb einzelner Abschnitte gleichbleibend oder sich kontinuierlich verändernd vorgegeben sein. Der Zuschnitt eines Funktionsabschnitts und insbesondere dessen Randabstand kann nach einer dreidimensionalen Erfassung des Rumpfes eines Trägers und nach einer Beurteilung des Krankheitsbildes einer Wirbelsäulenstellung mit Hilfe einer geeigneten Software ermittelt und vorgegeben werden.

Durch den in verschiedenen Richtungen eines Verlaufs eines Funktionsabschnitts unterschiedlich groß vorgegebenen Randabstand kann in der Richtung des Verlaufs eines Funktionsabschnitts und damit in der Richtung einer von der Orthese vorgegebenen Zugkraft eine möglichst große bzw. vorteilhafte Vorspannung des Funktionsabschnitts vorgegeben werden.

Es ist auch vorgesehen, dass der Rumpfabschnitt mit dem mindestens einen Halteabschnitt und dem mindestens einen Funktionsabschnitt einlagig ausgebildet ist. Um die den Tragekomfort weiter verbessernde Einlagigkeit zu ermöglichen und dennoch Kräfte und/oder Kraftmomente auf den Träger ausübend zu können ist der mindestens eine Funktionsabschnitt in den mindestens einen Halteabschnitt integriert, sodass keine störende Doppellagigkeit den Tragekomfort behindert. Weiterhin wird durch die Einlagigkeit ebenfalls die Herstellung des Rumpfabschnitts erleichtert, da keine mehrlagigen Bereiche mit relativ zueinander unterschiedlicher Vorspannung innerhalb eines mehrlagigen Bereichs mit übereinstimmender Vorspannung miteinander verbunden werden müssen. Dies könnte zu einem unterwünschten Faltenwurf des Rumpfabschnitts führen. Durch die einlagige Ausgestaltung hingegen kann ein Faltenwurf weitestgehend ausgeschlossen werden.

In einer Ausgestaltung der Erfindung ist es vorgesehen, dass der mindestens eine Funktionsabschnitt bei einer bestimmungsgemäßen Verwendung der Orthese den Rumpf des Trägers entlang einer Längsrichtung des Rumpfs zumindest abschnittweise schraubenförmig umgibt, und eine schraubenförmig verlaufende Zugkraft auf den Rumpf des Trägers ausübt. Neben einer Verkrümmung der Wirbelsäule durch eine Schieflage einzelner oder mehrere Wirbelkörper liegt bei einer Skoliose vielfach auch eine Verdrehung eines oder mehrerer Wirbelkörper vor. Eine derartige Verdrehung eines Wirbelkörpers resultiert oftmals in einer Verdrehung und/oder Verkippung von Schulter- und Hüftgürtel gegeneinander aus der Normallage. Durch eine schraubenförmigen Verlauf, zumindest aber durch einen sich über mehr als die Hälfte des Rumpfabschnitts erstreckenden diagonalen Verlaufs des mindestens einen Funktionsabschnitts entlang der Längsachse des Rumpfs können Kräfte bzw. Rotationskräfte auf den Rumpf übertragen werden, welche der Rotation der fehlgestellten Wirbelkörper und damit der Fehlstellung des Schulter- und Hüftgürtels entgegenwirken. Dabei ist der mindestens eine Funktionsabschnitt vorzugsweise so ausgebildet, dass es sich gegebenenfalls abwechselnd über die dreigeteilten Rumpfabschnitte Schultergürtel, Thorax und Pelvis und dabei auch sowohl über den vorderen Rumpf und den hinteren Rumpf erstreckt, sodass die entstehenden Zugkräfte der Verdrehung der Wirbelsäule entgegenwirken. Der mindestens eine Funktionsabschnitt verläuft dabei vorteilhafterweise bei einer bestimmungsgemäßen Verwendung der Orthese dabei im Wesentlichen weder vertikal noch horizontal, sondern diagonal.

Neben einem den Rumpf schraubenförmig umwindenden mindestens einen Funktionsabschnitt können auch mehrere Funktionsabschnitte so angeordnet werden, dass im Wesentlichen eine schraubenförmig resultierende Rotationskraft entsteht, die der unerwünschten Rotation der Wirbelkörper entgegenwirkt. Die Zugkräfte sind dabei vorzugsweise kontinuierlich entlang des schraubenförmig angeordneten mindestens einen Funktionsabschnitts angeordnet. Dies erzeugt bei jeder Drehung des Trägers eine gegenläufige Drehung, wodurch der stützende Muskelapparat zusätzlich angeregt wird.

Um einer seitlichen Verschiebung der Wirbelsäule und damit einer einhergehenden seitlichen Verschiebung des Schulter-und Hüftgürtels entgegenzuwirken ist es weiterhin optional auch vorgesehen, dass der Funktionsabschnitt auf einer Vorderseite und/oder auf einer Rückseite der Orthese v-förmig ausgebildet ist. Dabei ist es vorteilhaft, mindestens zwei Funktionsabschnitte so anzuordnen, dass eine gegenläufige durch die mindestens zwei Funktionsabschnitte bewirkte Zugkraft auf den Rumpf einwirkt. Vorteilhafterweise verläuft eine Zugrichtung dabei von einer Seite des Hüftgürtels schräg zu der gegenüberliegenden Seite der Taille sowie eine Zugrichtung von der Taille bis hin zu der gegenüberliegenden Seite des Schultergürtels.

Ein Vorteil der vorangehend beschriebenen Anordnung und Ausrichtung des mindestens einen Funktionsabschnitts wird in den Hebelkräften gesehen, die von dem mindestens einen Funktionsabschnitt erzeugt und auf einen Träger der Orthese übertragen werden. Die Endbereiche des mindestens einen Funktionsabschnitts oder der mehreren miteinander verbunden oder in Wirkverbindung stehenden Funktionsabschnitte sind dabei zweckmäßigerweise im Bereich des Schultergürtels und in der Nähe einer Hüfte angeordnet und über die in den Endbereichen angrenzenden Halteabschnitte an den betreffenden Bereichen des Rumpfes festgelegt. Durch den beispielsweise schraubenförmigen oder v-förmigen Verlauf, der sich jedenfalls diagonal über einen überwiegenden Abschnitt des Rumpfes erstreckt, werden nicht nur in einer unbewegten Haltung des Trägers, sondern insbesondere bei einer Bewegung des Trägers der Orthese ständig Hebelkräfte auf den Rumpf und die Extremitäten des Trägers ausgeübt, die während der Bewegung von dem Träger nicht als störend oder unangenehm empfunden werden, jedoch kontinuierlich einer Fehlstellung oder Fehlhaltung entgegenwirken. Auf diese Weise kann nicht nur den Auswirkungen einer Skoliose entgegengewirkt werden, sondern eine gesunde Haltung und Bewegung des Rumpfes für den Träger unterstützt und allmählich von dem Träger angelernt werden.

In einer weiteren Ausgestaltung des Erfindungsgedankens ist vorgesehen, dass sich der mindestens eine auf der Vorderseite angeordnete Funktionsabschnitt unmittelbar an einen Bereich des mindestens einen Funktionsabschnitts auf der Rückseite erstreckt. Dies ermöglicht es, die auf den Rumpf wirkenden Kräfte optimal kontrollieren zu können.

Es ist weiterhin auch vorgesehen, dass eine Richtung der maximalen Rückstellkraft des flexiblen textilen Gewebes des mindestens einen Funktionsabschnitts zumindest abschnittsweise dem schraubenförmigen Verlauf und/oder der V-Form folgt. In der Richtung der maximalen Rückstellkraft des flexiblen textilen Gewebes kann die größte Vorspannung und damit auch die größtmögliche resultierende Zugkraft ermöglicht werden. Die Ausrichtung der maximalen Rückstellkraft folgt dem Verlauf des Funktionsabschnitts, solange die Ausrichtung einen Winkel kleiner 15°, vorzugsweise kleiner 10° und besonders vorzugsweise kleiner 5° relativ zu dem Verlauf des Funktionsabschnitts aufweist, wobei der Verlauf des Funktionsabschnitts als längste mögliche Mittenlinie mit einem gleichen Abstand zu den gegenüberliegenden Randbereichen des Funktionsabschnitts definiert ist.

Einer weiteren optionalen Ausgestaltung des Erfindungsgedankens zufolge ist vorgesehen, dass ein Verhältnis des Randabstands zu einem zugeordneten Durchmesser des Zuschnitts des Funktionsabschnitts in dem nicht vorgespannten Zustand entlang des Umfangsrands zwischen 0,05 und 0,3, vorzugsweise zwischen 0,1 und 0,25 liegt. Es hat sich gezeigt, dass ein Randabstand in dieser Größenordnung in vielen Fällen eine Vorspannung des Funktionsabschnitts vorgibt, die einerseits von einem Träger noch als angenehm empfunden wird, andererseits jedoch eine therapeutisch wirksame Zugkraft und äußere Einwirkung auf den Träger der Orthese ausübt. Das Verhältnis des Randabstands zu dem zugeordneten Durchmesser des Zuschnitts des Funktionsabschnitts kann in Abhängigkeit der Rückstellkraft des Materials, aus welchem der betreffende Funktionsabschnitt hergestellt ist, angepasst und vorgegeben werden. Der zu einem Randabstand zugeordnete Durchmesser des Zuschnitts ist der Abstand zwischen den einander gegenüberliegenden Randbereichen des Zuschnitts entlang einer Geraden, die in gleicher Richtung wie der Randabstand verläuft, der zwischen zwei Punkten des Umfangsrands des Funktionsabschnitts in einem vorgespannten und in einem nicht vorgespannten Zustand gemessen wird.

In einer vorteilhaften Umsetzung des Erfindungsgedankens ist vorgesehen, dass das flexible textile Gewebe des Funktionsabschnitts und/oder des Halteabschnitts in unterschiedlichen Richtungen eine unterschiedliche Rückstellkraft aufweist. Durch die richtungsabhängigen Dehnungseigenschaften des flexiblen textilen Gewebes können die auf den Rumpf des Trägers bewirkten Kräfte präzise eingestellt werden, um die erwünschte und auf den Rumpf übertragene Zugkraft erreichen zu können. Eine weitere Möglichkeit, die resultierenden Zugkräfte präzise zu steuern, kann dadurch erreicht werden, dass das flexible textile Gewebe des Funktionsabschnitts und/oder des Halteabschnitts in unterschiedlichen Bereichen eine unterschiedliche Ausrichtung der Rückstellkraft aufweist.

Es ist auch vorgesehen, dass das elastische textile Gewebe des mindestens einen Funktionsabschnitts ein Gewebe aus Polyamid, Elastan, Ethylen-Ethylacrylat-Copolymer, oder ein Mischgewebe daraus aufweist. Durch die Verwendung von Kunstfasern kann der mindestens eine Funktionsbereich aus langlebigen und preisgünstig herzustellenden Fasern gebildet sein. Neben der Langlebigkeit können weiterhin auch die Eigenschaften des mindestens einen Funktionsbereichs präzise eingestellt werden, und die Eigenschaften des textilen Gewebes an die individuellen Ansprüche optimal angepasst werden.

Neben der Beeinflussung der Wirbelsäule kann es ebenfalls vorteilhaft sein, insbesondere um in geeigneter Weise auf eine aus einer Wirbelsäulenverkrümmung resultierenden Fehlstellung des Schulter- oder Hüftgürtels einwirken zu können, dass die Orthese auf die Gliedmaßen ausgeweitet wird. Daher ist es gemäß einer weiteren Ausgestaltung des Erfindungsgedankens auch vorgesehen, dass die Orthese einen an dem Rumpfabschnitt festgelegten Beinabschnitt und/oder einen Armabschnitt aufweist. Dies ermöglicht es, zusätzlich Kräfte auf die Gliedmaßen auszuüben und diese gegebenenfalls als Ankerpunkte für den mindestens einen Funktionsabschnitt nutzen zu können. Ein derartiger Armabschnitt und ein Beinabschnitt, die für die Kombination mit einem Rumpfabschnitt der erfindungsgemäß ausgestalteten Orthese besonders geeignet sind, sind beispielsweise in der Druckschrift DE 10 2016 108 151 A1 beschrieben.

Es ist weiterhin auch vorgesehen, dass der Rumpfabschnitt und/oder der Beinabschnitt und/oder der Armabschnitt ein Festlegungselement zur sicheren Festlegung des Rumpfabschnitts und/oder des Beinabschnitts und/oder des Armabschnitts an dem Träger aufweist. Auf diese Weise kann ein unerwünschtes Verrutschen eines der Abschnitte verhindert oder zumindest reduziert werden. Das Festlegungselement kann dafür als ein an dem Rumpfabschnitt und/oder dem Armabschnitt und/oder dem Beinabschnitt in Form eines Haftbands ausgebildet sein und durch eine erhöhte Haftwirkung bzw. Reibung zwischen dem Haftband und der Haut ein Verrutschen verhindern.

In einer vorteilhaften Ausgestaltung der Erfindung ist vorgesehen, dass eine sich von dem Rumpfabschnitt bis zu einem dem Rumpfbereich abgewandten Endbereich des Armabschnitts und/oder des Beinabschnitts erstreckende Mittenlinie des Armabschnitts und/oder des Beinabschnitts einen gekrümmten Verlauf aufweist. Wird der gekrümmte Armabschnitt und/oder der Beinabschnitt der Orthese von dem Träger verwendet, indem der Träger beispielsweise einen Arm in den gekrümmten Armabschnitt und/oder ein Bein in den gekrümmten Beinabschnitt einführt, wird der Armabschnitt und/oder der Beinabschnitt verformt und liegt eng an dem Arm oder Bein des Trägers an. Die Verformung des zunächst gekrümmten Armabschnitts und/oder des Beinabschnitts erzeugt Rückstellkräfte, die zusätzlich zu einer gegebenenfalls vorhandenen Kompressionswirkung während der Verwendung der Orthese weitere Kräfte oder Kraftmomente auf den Arm und/oder das Bein des Trägers ausüben.

Nachfolgend werden einige exemplarische Ausführungsbeispiele einer erfindungsgemäßen Ausgestaltung einer Orthese beschrieben, die in der Zeichnung dargestellt sind. Es zeigt:
Figur 1 eine schematische Vorderansicht sowie eine Rückenansicht eines Schnittbilds einer Orthese mit einem Rumpfabschnitt, wobei mehrere Funktionsabschnitte und mehrere Halteabschnitte erkennbar sind,
Figur 2 eine Ansicht eines Funktionsabschnitts aus Figur 1 mit eingezeichnetem Kraftvektor vor und nach dem Vernähen,
Figur 3 eine Ansicht eines weiteren Funktionsabschnitts aus Figur 1 mit eingezeichnetem Kraftvektor vor und nach dem Vernähen,
Figur 4 eine Ansicht eines weiteren Funktionsabschnitts aus Figur 1 mit eingezeichnetem Kraftvektor vor und nach dem Vernähen,
Figur 5 eine schematische Vorderansicht sowie eine Rückenansicht eines Schnittbilds einer Orthese mit einem Rumpfabschnitt, wobei jeweils ein Funktionsabschnitt v-förmig ausgebildet ist, und
Figur 6 eine schematische Vorderansicht und eine Rückenansicht einer abweichend ausgestalteten Orthese, mit welcher eine S-förmige Verkrümmung der Wirbelsäule in der Sagittalebene behandelt werden kann.

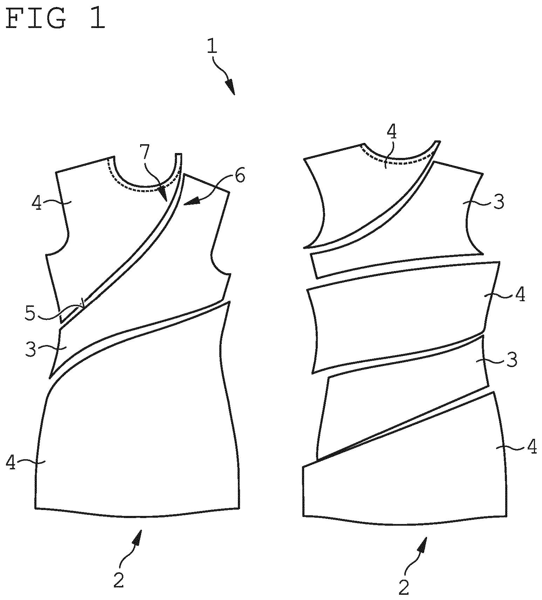

Figur 1 zeigt eine schematische Vorderansicht (links) sowie eine Rückenansicht (rechts) eines Schnittbilds einer Orthese 1 mit einem Rumpfabschnitt 2. Der Rumpfabschnitt 2 weist drei Funktionsabschnitte 3 und fünf Halteabschnitte 4 auf. Die Funktionsabschnitte 3 sowie die Halteabschnitte 4 sind dabei jeweils aus einem flexiblen textilen Gewebe hergestellt, wobei sich die jeweiligen textilen Gewebe der Funktionsabschnitte 3 und der Halteabschnitte 4 unterscheiden. Das textile Gewebe der Funktionsabschnitte 3 weist unter anderem eine größere Rückstellkraft als das textile Gewebe der Halteabschnitte 4 auf. Die Figur 1 zeigt dabei den Zustand der Orthese vor dem Vernähen der einzelnen Funktions- und Halteabschnitte 3, 4. Die Funktionsabschnitte 3 werden entlang eines Umfangsrands 5 mit jeweils einem Randbereich 6 der Funktionsabschnitte 3 mit einem Randbereich 7 eines Halteabschnitts entlang einer in Figur 1 nicht dargestellten Funktionsnaht miteinander verbunden. Weiterhin werden ebenfalls alle drei Funktionsabschnitte 3 miteinander ebenfalls über ihre Randbereiche 6 so miteinander über eine Funktionsnaht verbunden, sodass die miteinander verbundenen Funktionsbereiche 3 sich schraubenförmig entlang einer Längsachse des Rumpfabschnitts 2 und damit über einen Rumpf eines nicht näher dargestellten Trägers erstrecken.

Die miteinander zu verbindenden Randbereiche 6, 7 der aneinandergrenzenden Funktions- und Halteabschnitte 3, 4 weisen dabei eine unterschiedliche Länge auf, sodass bei einem Verbinden der beiden Randbereiche 6, 7 entlang der Funktionsnaht der Randbereich 6 des Funktionsabschnitts 3 gedehnt werden muss, bis beide Randbereiche 6, 7 gleich lang sind. Der vorgedehnte Randbereich 6 und damit der gegenüber dem noch nicht verbundenen und vorspannungsfreien Zuschnitt des betreffenden Funktionsabschnitts 3 wird damit mit einer Vorspannung versehen, woraus bei einem Anziehen der Orthese 1 und einer dadurch bewirkten weiteren Dehnung des Funktionsabschnitts 3 während der Verwendung der Orthese 1 die aus der Vorspannung resultierende Zugkraft auf den Träger übertragen wird. Durch die bewirkten Zugkräfte bzw. durch die aus der schraubenförmigen Anordnung auf die Wirbelsäule resultierenden Rotationskraft wird der Fehlstellung der Wirbelsäule eine Kraft entgegengesetzt. Durch die einlagige Ausbildung des Rumpfabschnitts 2 wird der Tragekomfort gesteigert und es kann einem vorzeitigen Therapieabbruch vorgebeugt werden.

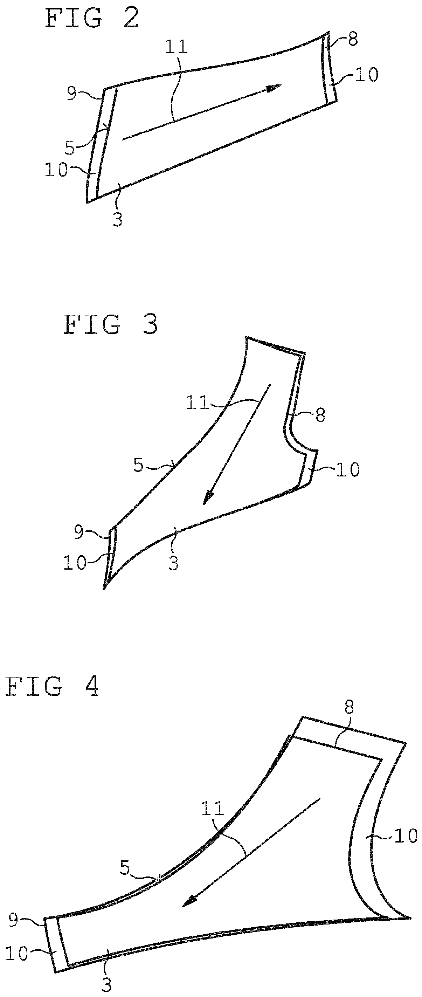

Die Figuren 2 bis 4 zeigen jeweils einen Funktionsabschnitt 3 aus Figur 1, wobei ein Zuschnitt des betreffenden Funktionsabschnitts 3 jeweils in seiner nicht gedehnten und deshalb nicht vorgespannten Form 8 sowie mit den Ausmaßen seiner gedehnten Form 9 dargestellt ist, die näherungsweise einem von den Zuschnitten der angrenzenden Halteabschnitten 4 nicht bedeckten Funktionsabschnittsbereich entspricht, in welchen der betreffende Funktionsabschnitt eingesetzt und mit der erforderlichen Dehnung und damit unter Vorspannung dann entlang seines Umfangsrands 5 mit den angrenzenden Halteabschnitten 4 verbunden wird. Der Zuschnitt des nicht gedehnten Funktionsabschnitts 3 bzw. der Form 8 weist gegenüber dem von den Halteabschnitten 4 freigelassenen Funktionsabschnittsbereich bzw. gegenüber der Form 9 in der Orthese 1 einen Randabstand 10 auf. Dieser Randabstand 10 kann entlang einiger Abschnitte des Umfangsrands 5 sehr klein sein oder den Wert Null betragen. Entlang anderer Abschnitte des Umfangsrands 5 und insbesondere in den Abschnitten, durch welche die gewünschten Zugkräfte der Orthese 1 verlaufen, kann der Randabstand 10 vergleichsweise groß sein und beispielsweise 10 bis 15 % eines gleichgerichtet verlaufenden Durchmessers des Funktionsabschnitts 3 betragen. Weiterhin zeigen die Figuren 2 bis 4 schematisch die aus der jeweiligen Vorspannung resultierende Zugkraft 11. Das textile Gewebe der einzelnen Funktionsabschnitte 3 ist zweckmäßigerweise jeweils so ausgerichtet, dass die Ausrichtung der maximalen Rückstellkraft in Richtung der aus der jeweiligen Vorspannung resultierenden Zugkraft 11 ausgerichtet ist. Dadurch kann die von dem Funktionsabschnitt 3 erzeugte Zugwirkung zusätzlich verstärkt werden und der Tragekomfort verbessert werden.

In der Figur 1 sind die einzelnen Funktionsabschnitte 3 jeweils in der nicht gedehnten und deshalb nicht vorgespannten Form 7 der jeweiligen Zuschnitte relativ zu den Zuschnitten der Halteabschnitte 4 gezeigt. Dadurch wird verdeutlicht, dass die Zuschnitte der Funktionsabschnitte 3 jeweils kleiner als die von den angrenzenden Halteabschnitten 4 freigelassenen Funktionsabschnittsbereiche sind, in welchen die Funktionsabschnitte 3 dann angeordnet und gedehnt und mit Vorspannung mit den angrenzenden Halteabschnitten 4 verbunden werden.

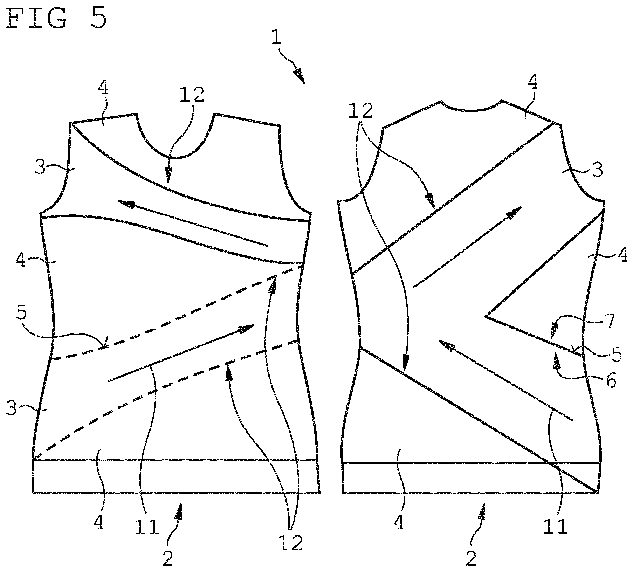

Die Figur 5 zeigt eine schematische Vorderansicht sowie eine Rückenansicht eines Schnittbilds einer Orthese 1 mit einem Rumpfabschnitt 2, wobei die Funktionsabschnitte 3 jeweils v-förmig ausgebildet sind. Die beiden Funktionsabschnitte 3 sind so angeordnet, dass eine durch den Funktionsabschnitte 3 bewirkte schraubenförmig verlaufende und gegenläufig gerichtete Zugkraft jeweils von dem Schultergürtel über einen mittleren Bereich des Thorax bis zu dem Pelvisbereich in der Nähe der Hüften auf den Rumpf einwirkt. Eine Richtung der Zugkraft 11 verläuft dabei von einer Seite des Hüftgürtels schräg zu der gegenüberliegenden Seite der Taille sowie eine weitere Richtung der Zugkraft 11 von der Taille bis hin zu der gegenüberliegenden Seite des Schultergürtels.

In Figur 5 sind auch die Verläufe von Funktionsnähten 12 dargestellt. Über die Funktionsnähte 12 sind aneinander angrenzende Bereiche der Funktionsabschnitte 3 und der Halteabschnitte 4 entlang des Umfangsrands 5 der Funktionsabschnitte 3 miteinander verbunden.

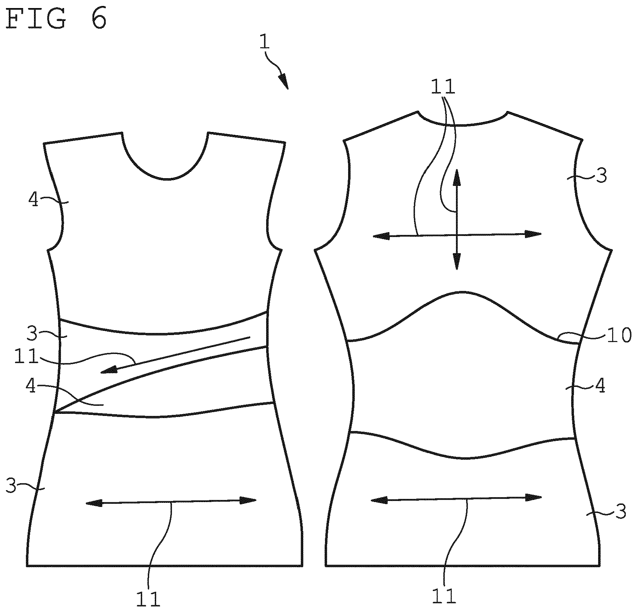

In Figur 6 ist schematisch eine abweichend ausgestaltete Orthese 1 dargestellt. Um einer S-förmigen Verkrümmung der Wirbelsäule in der Sagittalebene entgegenzuwirken ist auf der rechts dargestellten Rückseite der Orthese 1 ein beide Schulterbereiche überdeckender Funktionsabschnitt 3 angeordnet. Dieser Funktionsabschnitt 3 weist sowohl in horizontaler als auch in vertikaler Richtung eine große Rückstellkraft auf, sodass in allen Richtungen jeweils den Rücken eines Trägers in dessen Schulterbereich aufrichtende Zugkräfte 11 erzeugt werden. Die jeweils in der Nähe der Hüften angeordneten Funktionsabschnitte 3 sind in horizontaler Richtung vorgespannt und erzeugen dementsprechend in horizontaler Richtung verlaufende Zugkräfte 11. Auf der links dargestellten Vorderseite ist in einem mittleren Bereich ein diagonal verlaufender Funktionsabschnitt 3 eingearbeitet, der den Funktionsabschnitt 3 auf der Rückseite im Schulterbereich mit den Funktionsabschnitten 3 in der Nähe der Hüften verbindet und eine Verdrehung erzeugt. Zudem ist auf der Vorderseite eine Schulterhöhe des Halteabschnitts 4 auf einer Schulterseite um etwa 5 mm geringer als auf der anderen Schulterseite ausgestaltet. In dem Schulterbereich, an Ärmelsäumen und in Bündchen der Orthese 1 können rutschhemmende Einnäher eingenäht sein.

### BEZUGSZEICHENLISTE

- 1: Orthese
- 2: Rumpfabschnitt
- 3: Funktionsabschnitt
- 4: Halteabschnitt
- 5: Umfangsrand
- 6: Randbereich des Funktionsabschnitts
- 7: Randbereich des Halteabschnitts
- 8: Funktionsabschnitt in einer nicht gedehnten Form
- 9: Funktionsabschnitt in einer gedehnten Form
- 10: Randabstand
- 11: Zugkraft
- 12: Funktionsnaht

## Patentansprüche

1. Orthese (1) mit einem Rumpfabschnitt (2) zur Behandlung von Wirbelsäulenstellungen, die von einer Normalstellung abweichen, wobei der Rumpfabschnitt (2) bei einer bestimmungsgemäßen Verwendung der Orthese (1) einen Rumpf eines mit der Orthese (1) behandelnden Trägers zumindest abschnittsweise umhüllt, wobei der Rumpfabschnitt (2) mindestens einen aus einem ersten elastischen textilen Gewebe hergestellten Halteabschnitt (4) und mindestens einen ein zweites textiles Gewebe aufweisenden Funktionsabschnitt (3) aufweist, wobei der mindestens eine Funktionsabschnitt (3) eine Vorspannung aufweist, wobei eine aus der Vorspannung resultierende Kraft (11) und/oder ein Kraftmoment bei der bestimmungsgemäßen Verwendung der Orthese (1) auf den Rumpf des Trägers wirkt, wobei der mindestens eine Halteabschnitt (4) und der mindestens eine Funktionsabschnitt (3) überlappungsfrei miteinander verbunden sind und den Rumpfabschnitt (2) bilden, wobei ein Randbereich (6) des mindestens einen Funktionsabschnitts (3) mit einem Randbereich (7) des mindestens einen Halteabschnitts (4) entlang einer Funktionsnaht (10) miteinander verbunden ist, wobei die beiden längs der Funktionsnaht (12) miteinander verbundenen Randbereiche (6, 7) des mindestens einen Funktionsabschnitts (3) und des mindestens einen Halteabschnitts (4) eine voneinander abweichende Vorspannung aufweisen, und wobei ein Umfangsrand (5) eines Zuschnitts des mindestens einen Funktionsabschnitts (3) in einem nicht vorgespannten Zustand mindestens abschnittsweise einen Randabstand (10) zu einem Umfangsrand eines von den Zuschnitten der angrenzenden Halteabschnitten (4) nicht bedeckten Funktionsabschnittsbereichs aufweist, **dadurch gekennzeichnet, dass** der Randabstand (10) in Richtung eines Verlaufs des mindestens einen Funktionsabschnitts (3) größer, vorzugsweise um einen Faktor von 50% größer und besonders vorzugsweise um einen Faktor von 80% größer als in einer Richtung quer zu dem Verlauf des mindestens einen Funktionsabschnitts (3) ist, wobei der Verlauf des Funktionsabschnitts (3) als längste mögliche Mittenlinie mit einem gleichen Abstand zu den gegenüberliegenden Randbereichen (6) des Funktionsabschnitts (3) definiert ist.

2. Orthese (1) nach Anspruch 1, **dadurch gekennzeichnet, dass** der Rumpfabschnitt (2) mit dem mindestens einen Halteabschnitt (4) und dem mindestens einen Funktionsabschnitt (3) einlagig ausgebildet ist.

3. Orthese (1) nach Anspruch 1 oder 2, **dadurch gekennzeichnet, dass** der mindestens eine Funktionsabschnitt (3) bei einer bestimmungsgemäßen Verwendung der Orthese (1) den Rumpf des Trägers entlang einer Längsrichtung des Rumpfs zumindest abschnittweise schraubenförmig umgibt, und eine schraubenförmig verlaufende Zugkraft (9) auf den Rumpf des Trägers ausübt.

4. Orthese (1) nach einem der Ansprüche 1 bis 3, **dadurch gekennzeichnet, dass** der mindestens eine Funktionsabschnitt (3) auf einer Vorderseite und/oder auf einer Rückseite der Orthese v-förmig ausgebildet ist.

5. Orthese (1) nach Anspruch 4, **dadurch gekennzeichnet, dass** sich der mindestens eine auf der Vorderseite angeordnete Funktionsabschnitt (3) unmittelbar an einen Bereich des mindestens einen Funktionsabschnitts (3) auf der Rückseite erstreckt.

6. Orthese (1) nach Anspruch 3 oder 4, **dadurch gekennzeichnet, dass** eine Richtung der maximalen Rückstellkraft des elastischen textilen Gewebes des mindestens einen Funktionsabschnitts (3) dem zumindest abschnittsweise schraubenförmigen Verlauf und/oder der V-Form folgt.

7. Orthese (1) nach einem der vorangegangenen Ansprüche, **dadurch gekennzeichnet, dass** ein Verhältnis des Randabstands zu einem zugeordneten Durchmesser des Zuschnitts des Funktionsabschnitts (3) in dem nicht vorgespannten Zustand entlang des Umfangsrands zwischen 0,05 und 0,3, vorzugsweise zwischen 0,1 und 0,25 liegt.

8. Orthese (1) nach einem der vorangegangenen Ansprüche, **dadurch gekennzeichnet, dass** das elastische textile Gewebe des Funktionsabschnitts (3) und/oder des Halteabschnitts (4) in unterschiedlichen Richtungen eine unterschiedliche Rückstellkraft aufweist.

9. Orthese (1) nach einem der vorangegangenen Ansprüche, **dadurch gekennzeichnet, dass** das elastische textile Gewebe des Funktionsabschnitts (3) und/oder des Halteabschnitts (4) in unterschiedlichen Bereichen eine unterschiedliche Ausrichtung der Rückstellkraft aufweist.

10. Orthese (1) nach einem der vorangegangenen Ansprüche, **dadurch gekennzeichnet, dass** das elastische textile Gewebe des mindestens eine Funktionsabschnitt ein Gewebe aus Polyamid, Elastan, Ethylen-Ethylacrylat-Copolymer, oder ein Mischgewebe daraus aufweist.

11. Orthese (1) nach einem der vorangegangenen Ansprüche, **dadurch gekennzeichnet, dass** die Orthese (1) einen an dem Rumpfabschnitt (2) festgelegten Beinabschnitt und/oder einen Armabschnitt aufweist.

12. Orthese (1) nach Anspruch 11, **dadurch gekennzeichnet, dass** der Rumpfabschnitt (2) und/oder der Beinabschnitt und/oder der Armabschnitt ein Festlegungselement zur sicheren Festlegung des Rumpfabschnitts (2) und/oder des Beinabschnitts und/oder des Armabschnitts an dem Träger aufweist.

13. Orthese (1) nach Anspruch 11 oder 12, **dadurch gekennzeichnet, dass** sich eine von dem Rumpfabschnitt (2) bis zu einem dem Rumpfbereich (2) abgewandten Endbereich des Armabschnitts und/oder des Beinabschnitts erstreckende Mittenlinie des Armabschnitts und/oder des Beinabschnitts einen gekrümmten Verlauf aufweist.

## Claims

1. Orthosis (1) comprising a trunk portion (2) for treating spinal column positions which deviate from a normal position, wherein, when the orthosis (1) is used as intended, the trunk portion (2) at least partially surrounds a trunk of a wearer being treated with the orthosis (1), wherein the trunk portion (2) comprises at least one retaining portion (4) made from a first elastic textile fabric and at least one functional portion (3) having a second textile fabric, wherein the at least one functional portion (3) has a pre-tension, wherein a force (11) resulting from the pre-tension and/or a moment of force acts on the trunk of the wearer when the orthosis (1) is used as intended, wherein the at least one retaining portion (4) and the at least one functional portion (3) are connected to one another without overlap and form the trunk portion (2), wherein an edge region (6) of the at least one functional portion (3) is connected to an edge region (7) of the at least one retaining portion (4) along a functional seam (10), wherein the two edge regions (6, 7) of the at least one functional portion (3) and of the at least one retaining portion (4), which are connected to one another along the functional seam (12), have pre-tensions differing from one another, and wherein a peripheral edge (5) of a blank of the at least one functional portion (3), in a non-pre-tensioned state, has, over at least a portion thereof, an edge distance (10) from a peripheral edge of a region of the functional portion not covered by the blanks of the adjoining retaining portions (4), **characterised in that** the edge distance (10) is greater in a direction of a course of the at least one functional portion (3), preferably greater by a factor of 50% and particularly preferably greater by a factor of 80%, than in a direction transverse to the course of the at least one functional portion (3), wherein the course of the functional portion (3) is defined as the longest possible centre line having an equal distance from the opposite edge regions (6) of the functional portion (3).

2. Orthosis (1) according to claim 1, **characterised in that** the trunk portion (2), with the at least one retaining portion (4) and the at least one functional portion (3), is of single-layer construction.

3. Orthosis (1) according to claim 1 or 2, **characterised in that**, when the orthosis (1) is used as intended, the at least one functional portion (3) surrounds the trunk of the wearer helically, at least in portions, along a longitudinal direction of the trunk, and exerts on the trunk of the wearer a tensile force (9) having a helical course.

4. Orthosis (1) according to any one of claims 1 to 3, **characterised in that** the at least one functional portion (3) is formed in a V-shape on a front side and/or on a rear side of the orthosis.

5. Orthosis (1) according to claim 4, **characterised in that** the at least one functional portion (3) arranged on the front side extends directly to a region of the at least one functional portion (3) on the rear side.

6. Orthosis (1) according to claim 3 or 4, **characterised in that** a direction of the maximum restoring force of the elastic textile fabric of the at least one functional portion (3) follows the at least partially helical course and/or the V-shape.

7. Orthosis (1) according to any one of the preceding claims, **characterised in that** a ratio of the edge distance to an associated diameter of the blank of the functional portion (3) in the non-pre-tensioned state along the peripheral edge is between 0.05 and 0.3, preferably between 0.1 and 0.25.

8. Orthosis (1) according to any one of the preceding claims, **characterised in that** the elastic textile fabric of the functional portion (3) and/or of the retaining portion (4) has a different restoring force in different directions.

9. Orthosis (1) according to any one of the preceding claims, **characterised in that** the elastic textile fabric of the functional portion (3) and/or of the retaining portion (4) has a different orientation of the restoring force in different regions.

10. Orthosis (1) according to any one of the preceding claims, **characterised in that** the elastic textile fabric of the at least one functional portion comprises a fabric made of polyamide, elastane, ethylene-ethyl acrylate copolymer, or a blended fabric thereof.

11. Orthosis (1) according to any one of the preceding claims, **characterised in that** the orthosis (1) comprises a leg portion fixed to the trunk portion (2) and/or an arm portion.

12. Orthosis (1) according to claim 11, **characterised in that** the trunk portion (2) and/or the leg portion and/or the arm portion comprises a fixing element for securely fixing the trunk portion (2) and/or the leg portion and/or the arm portion to the wearer.

13. Orthosis (1) according to claim 11 or 12, **characterised in that** a centre line of the arm portion and/or of the leg portion, which extends from the trunk portion (2) to an end region of the arm portion and/or of the leg portion facing away from the trunk region (2), has a curved course.

## Revendications

1. Orthèse (1) comprenant une partie de tronc (2) destinée au traitement de positions de la colonne vertébrale qui s'écartent d'une position normale, dans laquelle, lorsque l'orthèse (1) est utilisée conformément à sa destination, la partie de tronc (2) enveloppe au moins partiellement le tronc d'un porteur traité au moyen de l'orthèse (1), dans laquelle la partie de tronc (2) comprend au moins une partie de maintien (4) réalisée à partir d'un premier tissu textile élastique et au moins une partie fonctionnelle (3) présentant un second tissu textile, dans laquelle l'au moins une partie fonctionnelle (3) présente une précontrainte, dans laquelle une force (11) résultant de la précontrainte et/ou un moment de force agit sur le tronc du porteur lorsque l'orthèse (1) est utilisée conformément à sa destination, dans laquelle l'au moins une partie de maintien (4) et l'au moins une partie fonctionnelle (3) sont reliées l'une à l'autre sans chevauchement et forment la partie de tronc (2), dans laquelle une zone de bord (6) de l'au moins une partie fonctionnelle (3) est reliée à une zone de bord (7) de l'au moins une partie de maintien (4) le long d'une couture fonctionnelle (10), dans laquelle les deux zones de bord (6, 7) de l'au moins une partie fonctionnelle (3) et de l'au moins une partie de maintien (4), reliées l'une à l'autre le long de la couture fonctionnelle (12), présentent des précontraintes différentes l'une de l'autre, et dans laquelle un bord périphérique (5) d'une pièce découpée de l'au moins une partie fonctionnelle (3), dans un état non précontraint, présente au moins par endroits une distance au bord (10) par rapport à un bord périphérique d'une zone de la partie fonctionnelle non couverte par les pièces découpées des parties de maintien adjacentes (4), **caractérisée en ce que** la distance au bord (10) est plus grande dans une direction d'un tracé de l'au moins une partie fonctionnelle (3), de préférence d'un facteur de 50 % et, de manière particulièrement préférée, d'un facteur de 80 %, que dans une direction transversale au tracé de l'au moins une partie fonctionnelle (3), le tracé de la partie fonctionnelle (3) étant défini comme la plus longue ligne médiane possible ayant une distance égale par rapport aux zones de bord opposées (6) de la partie fonctionnelle (3).

2. Orthèse (1) selon la revendication 1, **caractérisée en ce que** la partie de tronc (2), avec l'au moins une partie de maintien (4) et l'au moins une partie fonctionnelle (3), est réalisée en une seule couche.

3. Orthèse (1) selon la revendication 1 ou 2, **caractérisée en ce que**, lorsque l'orthèse (1) est utilisée conformément à sa destination, l'au moins une partie fonctionnelle (3) entoure le tronc du porteur de manière hélicoïdale, au moins par endroits, le long d'une direction longitudinale du tronc, et exerce sur le tronc du porteur une force de traction (9) suivant un tracé hélicoïdal.

4. Orthèse (1) selon l'une quelconque des revendications 1 à 3, **caractérisée en ce que** l'au moins une partie fonctionnelle (3) est réalisée en forme de V sur une face avant et/ou sur une face arrière de l'orthèse.

5. Orthèse (1) selon la revendication 4, **caractérisée en ce que** l'au moins une partie fonctionnelle (3) disposée sur la face avant s'étend directement jusqu'à une zone de l'au moins une partie fonctionnelle (3) située sur la face arrière.

6. Orthèse (1) selon la revendication 3 ou 4, **caractérisée en ce qu'**une direction de la force de rappel maximale du tissu textile élastique de l'au moins une partie fonctionnelle (3) suit le tracé au moins partiellement hélicoïdal et/ou la forme en V.

7. Orthèse (1) selon l'une quelconque des revendications précédentes, **caractérisée en ce qu'**un rapport de la distance au bord à un diamètre associé de la pièce découpée de la partie fonctionnelle (3), dans l'état non précontraint, le long du bord périphérique, est compris entre 0,05 et 0,3, de préférence entre 0,1 et 0,25.

8. Orthèse (1) selon l'une quelconque des revendications précédentes, **caractérisée en ce que** le tissu textile élastique de la partie fonctionnelle (3) et/ou de la partie de maintien (4) présente une force de rappel différente dans des directions différentes.

9. Orthèse (1) selon l'une quelconque des revendications précédentes, **caractérisée en ce que** le tissu textile élastique de la partie fonctionnelle (3) et/ou de la partie de maintien (4) présente, dans des zones différentes, une orientation différente de la force de rappel.

10. Orthèse (1) selon l'une quelconque des revendications précédentes, **caractérisée en ce que** le tissu textile élastique de l'au moins une partie fonctionnelle comprend un tissu en polyamide, en élasthanne, en copolymère éthylène-acrylate d'éthyle, ou un tissu mélangé à base de ceux-ci.

11. Orthèse (1) selon l'une quelconque des revendications précédentes, **caractérisée en ce que** l'orthèse (1) comprend une partie de jambe fixée à la partie de tronc (2) et/ou une partie de bras.

12. Orthèse (1) selon la revendication 11, **caractérisée en ce que** la partie de tronc (2) et/ou la partie de jambe et/ou la partie de bras comprend un élément de fixation destiné à fixer de manière sûre la partie de tronc (2) et/ou la partie de jambe et/ou la partie de bras au porteur.

13. Orthèse (1) selon la revendication 11 ou 12, **caractérisée en ce qu'**une ligne médiane de la partie de bras et/ou de la partie de jambe, s'étendant de la partie de tronc (2) jusqu'à une zone d'extrémité de la partie de bras et/ou de la partie de jambe orientée à l'opposé de la zone de tronc (2), présente un tracé courbe.
ed course.
